# EUROPEAN PATENT SPECIFICATION

(11) **EP 4 444 696 B1**
(45) Date of publication and mention of the grant of the patent: **26.11.2025**
(21) Application number: 22847645.3
(22) Date of filing: 05.12.2022
(51) Int. Cl.: C07C 309/31, C07C 303/08

(54) **PRODUCTION PROCESS FOR 3,5-DI-TERT-BUTYLBENZENESULFONIC ACID**
VERFAHREN ZUR HERSTELLUNG VON 3,5-DI-TERT-BUTYLBENZOLSULFONSÄURE
PROCÉDÉ DE PRODUCTION D'ACIDE 3,5-DI-TERT-BUTYLBENZÈNESULFONIQUE

(30) Priority: 06.12.2021 US 202163286278 P
(43) Date of publication of application: 16.10.2024
(73) Proprietor: The Chemours Company FC, LLC, Wilmington, Delaware 19801 (US)
(72) Inventor: MORKEN, Peter A., Wilmington, Delaware 19810 (US); ZIMMERMAN, William H., Wilmington, Delaware 19808 (US); CAREY, Edward Patrick, Atglen, Pennsylvania 19310 (US); MATSNEV, Andrii, The Plains, Ohio 45780 (US)
(74) Representative: Abitz & Partner
(86) International application number: PCT/US2022/051768
(87) International publication number: WO 2023/107354

(56) References cited:
- WO-A1-02/30878
- RIS ET AL.: "Aromatic sulfonation. Ll. Sulfonation of 1,3,5-tri- and m- and p-di-tert-butylbenzene, the three tert-butylbenzenesulfonic acids, and 3,5-di-tert-butylbenzenesulfonic acid", JOURNAL OF THE CHEMICAL SOCIETY, PERKIN TRANSACTIONS 2: PHYSICAL ORGANIC CHEMISTRY, vol. 13, 1975, pages 1438 - 1445, XP093032548

## Description

### FIELD OF THE INVENTION

This invention relates to a production process for 3,5-di-tert-butylbenzenesulfonic acid and more particularly relates to a production process for 3,5-di-tert-butylbenzenesulfonic acid that provides for recovery of high purity 3,5-di-tert-butylbenzenesulfonic acid without expensive purification steps.

### BACKGROUND OF THE INVENTION

3,5-Di-tert-butylbenzenesulfonic acid is useful as a non-fluorinated surfactant for fluoropolymer emulsion polymerization processes due to its low reactivity during polymerization and good dispersion stabilizing properties. As reported by Ris et al. ("Aromatic sulfonation. LI. Sulfonation of 1,3,5-tri- and m- and p-di-tert-butylbenzene, the three tert-butylbenzenesulfonic acids, and 3,5-di-tert-butylbenzenesulfonic acid", Journal of the Chemical Society, Perkin Transactions 2: Physical Organic Chemistry, Vol. 13, pp. 1438-1445 (1975), 3,5-di-tert-butylbenzenesulfonic acid can be prepared by sulfonation of 1,3-di-tert-butylbenzene. 1,3-di-tert-butylbenzene, however, is an expensive starting material. Ris also describes how 3,5-di-tert-butylbenzenesulfonic acid can alternatively be obtained by dealkylative sulfonation of lower-cost starting material 1,3,5-tri-tert-butylbenzene (TTBB) with sulfur trioxide. This alternative process, however, has the disadvantage of the significant formation of side-products such as 2-methylene-1,3-propanedisulfonic acid (PDSA) and thus requires expensive purification steps.

### SUMMARY OF THE INVENTION

The invention, in part, is based on the discovery that exposing a solution of 1,3,5-tri-tert-butyl benzene (TTBB) in a solvent to anhydrous hydrogen chloride gas prior to reaction with a sulfonating agent to produce 3,5-di-tert-butylbenzenesulfonic acid suppresses the formation of 1,3-propanedisulfonic acid, 2-methylene- (PDSA) as a side product. A process for producing 3,5-di-tert-butylbenzenesulfonic acid in accordance with the invention comprises:
forming a solution of 1,3,5-tri-tert-butyl benzene in a solvent;
exposing the solution to anhydrous hydrogen chloride gas;
reacting 1,3,5-tri-tert-butylbenzene with a sulfonating agent to form 3,5-di-tert-butylbenzenesulfonic acid;
   wherein the exposing of the solution to anhydrous hydrogen chloride gas is carried out before reacting the 1,3,5-tri-tert-butylbenzene with the sulfonating agent; and
recovering the 3,5-di-tert-butylbenzenesulfonic acid.

In one embodiment of the process, the solvent is selected to provide solubility for the amount of 1,3,5-tri-tert-butyl benzene used.

In another embodiment of the process, the solvent is selected to provide solubility for the amounts of 1,3,5-tri-tert-butyl benzene and the sulfonating agent used.

In another embodiment of the process, the solvent is selected to provide that 3,5-di-tert-butylbenzenesulfonic acid is substantially insoluble in the solvent.

In another embodiment of the process, the 3,5-di-tert-butylbenzenesulfonic acid is recovered by filtration.

In another embodiment of the process, the solvent is an organic solvent.

In another embodiment of the process, the organic solvent is dichloromethane.

In another embodiment of the process, the sulfonating agent is chlorosulfonic acid or sulfur trioxide.

In another embodiment of the process, the exposing of the solution to anhydrous hydrogen chloride gas and the reacting the 1,3,5-tri-tert-butyl benzene with a sulfonating agent is carried out at a temperature of about -50°C to about 10°C.

In another embodiment of the process, the exposing of the solution to anhydrous hydrogen chloride gas is carried out under pressure.

In another embodiment of the process, the exposing of the solution to anhydrous hydrogen chloride gas is carried out to substantially saturate the solution with anhydrous hydrogen chloride.

In another embodiment of the process, the exposing of the solution to anhydrous hydrogen chloride gas is carried out by injecting the anhydrous hydrogen chloride gas into the solution.

Because the process of the invention suppresses the formation of 1,3-propanedisulfonic acid, 2-methylene- (PDSA) as a side product, 3,5-di-tert-butylbenzenesulfonic acid can be recovered without expensive purification steps to remove PDSA. For example, in the embodiment of the process in which the solvent is selected to provide for substantial insolubility of the 3,5-di-tert-butylbenzenesulfonic acid, high purity 3,5-di-tert-butylbenzenesulfonic acid can be recovered by filtration.

### DETAILED DESCRIPTION OF THE INVENTION

The starting material for the process, 1,3,5-tri-tert-butyl benzene (TTBB), suitable for the practice of the present invention, is available commercially.

Any of a variety of sulfonating agents can be used that can react with the 1,3,5-tri-tert-butylbenzene by dealkylative sulfonation to form 3,5-di-tert-butylbenzenesulfonic acid. Suitable sulfonating agents may include, but are not limited to, chlorosulfonic acid, sulfur trioxide, concentrated sulfuric acid, oleum, fluorosulfonic acid, and/or sulfur trioxide-Lewis base complexes. Preferably, the sulfonating agent is chlorosulfonic acid or sulfur trioxide, most preferably chlorosulfonic acid.

The process comprises forming a solution of 1,3,5-tri-tert-butyl benzene in a solvent. In one embodiment of the process, the solvent is selected to provide solubility for the amount of 1,3,5-tri-tert-butyl benzene used. In another embodiment of the process, the solvent is selected to provide solubility for the amounts of 1,3,5-tri-tert-butyl benzene and the sulfonating agent used. It is not necessary for the solvent to be miscible with the 1,3,5-tri-tert-butyl benzene or the sulfonating agent. The solvent selected preferably provides sufficient solubility for the amount of 1,3,5-tri-tert-butyl benzene employed, and preferably also the amount of sulfonating agent employed, so that the dealkylative sulfonation to form 3,5-di-tert-butylbenzenesulfonic acid can be carried out effectively.

In another embodiment of the process, the solvent is selected to provide that 3,5-di-tert-butylbenzenesulfonic acid is substantially insoluble in the solvent. This embodiment of the process is advantageous, since it facilitates recovery of the 3,5-di-tert-butylbenzenesulfonic acid product and enables another embodiment of the invention, i.e., recovery of the 3,5-di-tert-butylbenzenesulfonic acid by filtration.

In some embodiments, the solvent is an inorganic solvent or an organic solvent.

In another embodiment of the process, the solvent is an inorganic solvent. Appropriate inorganic solvents may include, but are not limited to, liquified sulfur dioxide.

In another embodiment of the process, the solvent is an organic solvent. Examples of organic solvents that provide solubility for the amount of 1,3,5-tri-tert-butyl benzene employed and also the amount of sulfonating agent, while providing substantial insolubility for the 3,5-di-tert-butylbenzenesulfonic acid, include chloroalkanes. In another embodiment of the process, the organic solvent is dichloromethane (DCM). Other appropriate organic solvents may include, but are not limited to, liquified sulfur dioxide, t-butyl chloride, or a mixture of t-butyl chloride and DCM or another chlorinated solvent.

In another embodiment of the process, the ratio of TTBB to solvent in the initial solution is in the range of, by weight, about 3 to about 30, preferably about 4 to about 20, more preferably about 5 to about 10.

The process further comprises exposing the solution to anhydrous hydrogen chloride gas.

The exposing may occur at any of a range of pressure conditions, such as, for example, about 90 kPa (absolute) to about 700 kPa (absolute) pressure. As the exposing pressure increases, more hydrogen chloride can be added to the solution to increase the suppression 1,3-propanedisulfonic acid, 2-methylene- (PDSA) formation. In some embodiments, the exposing pressure is selected to provide a predetermined level of hydrogen chloride in the solution.

In some embodiments of the process, the exposing occurs at lower pressure conditions in the range of about 90 kPa (absolute) to about 110 kPa (absolute), preferably about 95 kPa (absolute) to about 105 kPa (absolute), more preferably about 100 kPa (absolute) to about 103 kPa (absolute), or about atmospheric pressure.

In other embodiment of the process, the exposing of the solution to anhydrous hydrogen chloride gas is carried out under higher pressure conditions. The higher pressure may be achieved by sealing the reactor and adding pressurized anhydrous hydrogen chloride gas until the higher pressure is achieved. Appropriate ranges of higher pressure include in the range of about 110 kPa (absolute) to about 700 kPa (absolute), alternatively in the range of about 150 kPa (absolute) to about 400 kPa (absolute), alternatively in the range of about 200 kPa (absolute) to about 250 kPa (absolute), alternatively in the range of about 400 kPa (absolute) to about 700 kPa (absolute), or any value, range, or sub-range therebetween.

In another embodiment of the process, the exposing of the solution to anhydrous hydrogen chloride gas is carried out to substantially saturate the solution with anhydrous hydrogen chloride.

In another embodiment of the process, the exposing of the solution to anhydrous hydrogen chloride gas is carried out by injecting the anhydrous hydrogen chloride gas into the solution.

The exposing of the solution to anhydrous hydrogen chloride gas is carried out before reacting the 1,3,5-tri-tert-butylbenzene with a sulfonating agent. The exposing to anhydrous hydrogen chloride gas preferably continues through the reacting with the sulfonating agent.

After the solution has been exposed to anhydrous hydrogen chloride gas, the process further comprises reacting the 1,3,5-tri-tert-butylbenzene with the sulfonating agent to form 3,5-di-tert-butylbenzenesulfonic acid. In some embodiments, the amount of sulfonating agent is equimolar or substantially equimolar with the amount of 1,3,5-tri-tert-butylbenzene present.

In another embodiment of the process, the sulfonating agent is added dropwise to the solution.

In another embodiment of the process, the sulfonating agent is added subsurface to the solution by way of a dip tube.

In another embodiment of the process, the sulfonating agent is added to the solution over a time period in the range of five minutes to six hours.

In another embodiment of the process, the reacting occurs over a time period in the range of about four hours to about six hours.

In another embodiment of the process, the exposing of the solution to anhydrous hydrogen chloride gas and the reacting the 1,3,5-tri-tert-butyl benzene with a sulfonating agent is carried out at a temperature in the range of about -50°C to about 10°C, preferably in the range of about -40°C to about -10°C, more preferably in the range of about -30°C to about -20°C.

The process further comprises recovering the 3,5-di-tert-butylbenzenesulfonic acid.

In another embodiment of the process, the recovering includes cold filtering the reaction mixture, washing the solid product with cold organic solvent, and drying the solid product.

In another embodiment of the process, the byproduct t-butyl chloride substantially remains in the liquid phase and is substantially separated from the solid product by the filtering and washing.

The yield of the reaction is at least 50%, preferably at least 60%, more preferably at least 70%.

In another embodiment of the process, the purity of the 3,5-di-tert-butylbenzenesulfonic acid after the filtering, washing, and drying is determined. In some embodiments, the purity of the 3,5-di-tert-butylbenzenesulfonic acid is determined by proton nuclear magnetic resonance (¹H NMR). In other embodiments, the purity of the 3,5-di-tert-butylbenzenesulfonic acid may be determined by liquid chromatography-mass spectrometry (LC-MS). In yet other embodiments, the purity of the 3,5-di-tert-butylbenzenesulfonic acid may be determined by high performance liquid chromatography (HPLC).

In another embodiment of the process, the amount of PDSA in the solid product is less than 2 mol%, preferably less than 1 mol%, more preferably less than 0.2 mol%. In some embodiments of the process, the organic purity of the 3,5-di-tert-butylbenzenesulfonic acid in the solid product after the filtering, washing, and drying is at least 95%, preferably at least 98%, more preferably at least 99%. As used herein, organic purity refers to the purity of the 3,5-di-tert-butylbenzenesulfonic acid with respect to the presence of PDSA and any related sulfonated compounds and oligomers.

In another embodiment of the process, the solid product is purified by recrystallization in a solvent. Preferably the solvent is dichloromethane.

In another embodiment of the process, a reagent is added to quench unreacted sulfonating agent prior to the recovering. In some embodiments, the reagent is water.

In another embodiment of the process, hydrogen chloride is removed from the solution prior to the recovering. In some embodiments, substantially all of the hydrogen chloride is removed from the solution prior to the recovering.

In another embodiment of the process, a stoichiometric amount of water is added prior to the recovering such that the product is collected as the monohydrate of 3,5-di-tert-butylbenzenesulfonic acid.

Because the process of the invention suppresses the formation of 1,3-propanedisulfonic acid, 2-methylene- (PDSA), 3,5-di-tert-butylbenzenesulfonic acid can be recovered without expensive purification steps to remove PDSA. For example, in the embodiment of the process in which the solvent is selected to provide for substantial insolubility of the 3,5-di-tert-butylbenzenesulfonic acid, high purity 3,5-di-tert-butylbenzenesulfonic acid can be recovered by filtration.

### TEST METHODS

The product purity was determined by ¹H NMR. After filtering, washing, and drying, the product was dissolved in DMSO-d6 and an ¹H NMR spectrum was collected. The product purity was calculated based on the relative areas of the peaks for the 3,5-di-tert-butylbenzenesulfonic acid (600 MHz, DMSO-d6) δ 7.47 (d, 2 Hz, 2H), 7.39 (t, 2Hz, 1H), 1.26 (s, 18H) and the PDSA δ 5.11 (s, 1H), 3.60 (s, 2H).

### EXAMPLES

### Comparative Example 1

3,5-Di-tert-butylbenzenesulfonic acid was produced on a small scale following Scheme 1 without exposure to anhydrous HCl prior to reaction with the sulfonating agent chlorosulfonic acid.

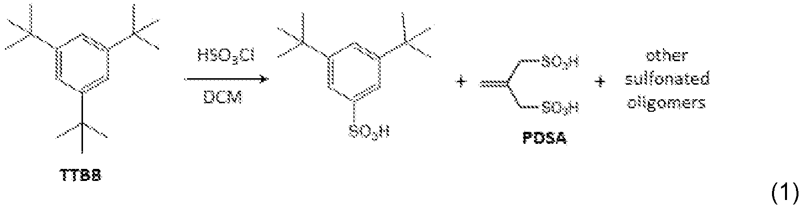

A jacketed 500-mL flask was equipped with a stir bar, a sub-surface nitrogen inlet, a thermocouple, a dropping funnel, and a dry ice condenser vented to a caustic scrubber. Check valves were installed before the gas inlet tube and scrubber. The flask was charged with 1,3,5-tri-tert-butyl benzene (25.09 g, 0.102 mol) and 125 mL of dry dichloromethane (DCM, CH₂Cl₂). The solution was chilled to -10°C, then chlorosulfonic acid (12.3 g, 0.105 mol) was added dropwise over a 15-minute period. The mixture was stirred for 4 hours while bubbling nitrogen (N₂) through the reaction mass, then water (0.15 g, 8.3 mmol) was added and nitrogen was bubbled through the mixture for an additional 30 minutes. The mixture was filtered while cold through a polypropylene filter funnel with a 10-µm polyethylene fritted disc and washed with 150 mL of chilled (-40°C) dichloromethane. The solids were dried at 40°C/40 mm Hg to afford 15.2 g (0.056 mol, 55% yield) of an off-white solid product.

The product composition was determined by ¹H NMR. The main constituent was 3,5-di-tert-butylbenzenesulfonic acid. The ¹H NMR also revealed that the product composition contained 17.4 mol% of 1,3-propanedisulfonic acid, 2-methylene- (PDSA, CAS # 1561-93-9).

The 3,5-di-tert-butylbenzenesulfonic acid product containing PDSA required an additional purification step to provide a high purity product. For example, 3,5-di-tert-butylbenzenesulfonic acid product containing PDSA may be purified by recrystallization from dichloromethane to reduce the PDSA concentration to <0.1 mol%.

An orange, sticky, insoluble residue remained in the flask. It was believed to contain sulfonated isobutylene oligomers.

The filtrate was concentrated by rotary evaporator and the distillate was analyzed by gas chromatography with a flame ionization detector (FID). The area of the t-butyl chloride peak divided by the area of the dichloromethane peak was 0.054.

### Inventive Example 1

3,5-Di-tert-butylbenzenesulfonic acid was produced on a small scale following Scheme 2 with exposure to anhydrous HCl under lower pressure conditions prior to reaction with the sulfonating agent chlorosulfonic acid.

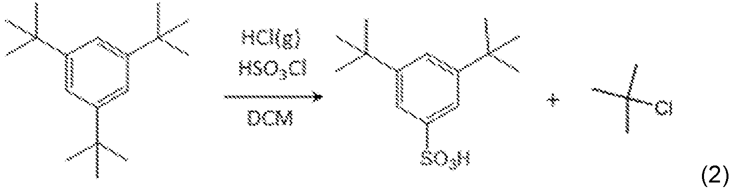

A jacketed 500-mL flask was equipped with a stir bar, a thermocouple, a sub-surface gas inlet tube, a dropping funnel, and a dry ice condenser vented to a caustic scrubber. Check valves were installed before the gas inlet tube and scrubber. The flask was charged with 1,3,5-tri-tert-butyl benzene (25.44 g, 0.103 mol) and 125 mL of dry dichloromethane. The solution was chilled to -10°C, then anhydrous HCl gas was introduced at atmospheric pressure through the gas inlet tube for a period of 10 minutes to substantially saturate the dichloromethane solution. Chlorosulfonic acid (12.3g, 0.105 mol) was added dropwise over a 7-minute time period. The mixture was stirred for 4 hours while continuing to bubble HCl though the reaction mass, then water (0.15g, 8.3 mmol) was added and nitrogen (N₂) was bubbled through the mixture for 30 minutes. A total of 18.9 g (0.517 moles) of anhydrous HCl was added during the process. The mixture was filtered while cold through a polypropylene filter funnel with a 10-µm polyethylene fritted disc and washed with 150 mL of chilled (-40°C) dichloromethane. The solids were dried at 40°C/40 mm Hg to afford 19.8 g (0.073 mol, 71 % yield) of an off-white solid product.

The product composition was determined by ¹H NMR. The main constituent was 3,5-di-tert-butylbenzenesulfonic acid. The ¹H NMR also revealed that the product composition contained only 1.5 mol% of PDSA.

The aryl sulfonic acid product may be further purified by, for example, recrystallization from dichloromethane to reduce the PDSA concentration, for example, to less than 0.1 mol%.

No orange, sticky, insoluble residue remained in the flask, indicating that introducing excess anhydrous HCl into the system suppressed the formation of sulfonated isobutylene oligomers.

The filtrate was concentrated by rotary evaporator and the distillate was analyzed by gas chromatography with FID. The area of the t-butyl chloride peak divided by the area of the dichloromethane peak was 0.10, indicating approximately twice as much t-butyl chloride was formed when excess anhydrous HCl is introduced into the system.

### Comparative Example 2

3,5-Di-tert-butylbenzenesulfonic acid was produced on a larger scale than Comparative Example 1 following Scheme 1 without exposure to anhydrous HCl prior to reaction with the sulfonating agent chlorosulfonic acid.

A jacketed 5-L reactor was equipped with overhead stirring, a thermocouple, a gas inlet tube, a dropping funnel, and a dry ice condenser vented to a caustic scrubber. Check valves were installed before the gas inlet tube and scrubber. The flask was charged with 1,3,5-tri-tert-butyl benzene (256 g, 1.04 mol, PHT International Inc., Charlotte, NC) and 1000 mL of dry dichloromethane. The solution was chilled to -10 to -13°C, then nitrogen (N₂) was introduced through the gas inlet tube for a period of 30 minutes to make the headspace inert and purge dissolved gases. Subsurface introduction of nitrogen was discontinued and chlorosulfonic acid (125 g, 1.07 mol) was added dropwise over a 30-minute period of time. The mixture was stirred for 4 hours, then water (31 g, 2.16 mol) was added and nitrogen was bubbled through the mixture for 40 minutes to remove any dissolved HCl. The mixture was filtered while cold through a filter funnel with a 45-90 µm high-density polyethylene (HDPE) frit and washed with 2000 mL of chilled dichloromethane. The solids were dried at ambient temperature under a stream of nitrogen to afford 110 g (0.407 mol, 39% yield) of a light-pink solid.

The product composition was determined by ¹H NMR. The main constituent was 3,5-Di-tert-butylbenzenesulfonic acid. The ¹H NMR also revealed that the product composition contained 30.8 mol% of PDSA.

The 3,5-di-tert-butylbenzenesulfonic acid product containing PDSA required an additional purification step to provide a high purity product. For example, 3,5-di-tert-butylbenzenesulfonic acid product containing PDSA may be purified by recrystallization from dichloromethane to reduce the PDSA concentration, for example, to <0.1 mol%.

A reddish-orange sticky, insoluble residue remained on the reactor walls and agitator. The residue was believed to contain sulfonated isobutylene oligomers.

### Inventive Example 2

3,5-Di-tert-butylbenzenesulfonic acid was produced on a larger scale than Inventive Example 1 following Scheme 2 with exposure to anhydrous HCl under lower pressure conditions prior to reaction with the sulfonating agent chlorosulfonic acid.

A jacketed 5-L reactor was equipped with an overhead stirrer, a gas inlet tube, a thermocouple, a dropping funnel, and a dry ice condenser vented to a caustic scrubber. Check valves were installed before the gas inlet tube and scrubber. The flask was charged with 1,3,5-tri-tert-butyl benzene (750 g, 3.04 mol, PHT International Inc.) and 3.125 L of dry dichloromethane. The solution was chilled to - 10 to -13°C, then anhydrous HCl gas was introduced through the gas inlet tube for a period of 1 hour at a vigorous rate to saturate the dichloromethane solution. The HCl addition rate was then slowed, and chlorosulfonic acid (365 g, 3.13 mol) was added dropwise over a 40-minute period. The mixture was stirred for 4 hours, then water (13.5 g, 0.75 mol) was added and nitrogen (N₂) was bubbled through the mixture for 1.5 hours. A total of 191 g (5.24 moles) of anhydrous HCl was added to the process. The mixture was filtered while cold through a filter funnel with a 45-90-µm HDPE frit and washed with 2 L of chilled (-40°C) dichloromethane. The solids were dried at room temperature on the filter with vacuum and under a stream of nitrogen to afford 587 g (2.17 mol, 71% yield) of an off-white solid product.

The product composition was determined by ¹H NMR. The main constituent was 3,5-di-tert-butylbenzenesulfonic acid. The ¹H NMR also revealed that the product composition contained only 0.1 mol% PDSA.

The aryl sulfonic acid product may be further purified by, for example, recrystallization from dichloromethane to reduce the PDSA concentration, for example, to less than 0.05 mol%.

No orange, sticky, insoluble residue remains in the flask, indicating that introducing excess anhydrous HCl into the system suppresses the formation of sulfonated isobutylene oligomers.

### Inventive Example 3

3,5-Di-tert-butylbenzenesulfonic acid was produced on a large-scale following Scheme 2 with exposure to anhydrous HCl under higher pressure conditions prior to reaction with the sulfonating agent chlorosulfonic acid.

29 kg of 1,3,5-tri-tert-butylbenzene solid was charged to a nominal 200-gal glass-lined reactor. The reactor was then sealed, made inert with nitrogen, and then evacuated to 20 kPa (absolute) pressure. 270 kg of dichloromethane was pumped to the reactor. With agitation, full cooling was applied to the reactor through an external jacket. The reactor and its contents were cooled to -26°C. The 1,3,5-tri-tert-butylbenzene was fully soluble in dichloromethane at this temperature.

Gaseous, anhydrous hydrogen chloride was feed to the reactor from a cylinder subsurface into the solution through a dip tube. Over a period of 6 hours, 12.5 kg of anhydrous HCl was added to the reactor, increasing the reactor pressure to 120 kPa.

14.5 kg of chlorosulfonic acid was fed to the reactor, subsurface into the solution through a dip tube, over a period of 6 hours. The addition of chlorosulfonic acid was exothermic and the temperature of the reactor increased slightly. After the chlorosulfonic acid feed was complete, the reactor was allowed to cool down. During this period a 3,5-di-tert-butylbenzenesulfonic acid precipitate formed in the reactor.

0.63 kg of deionized water was added to the reactor from the top of the reactor above the surface of the liquid to quench any unreacted chlorosulfonic acid. The 0.63 kg represents about 28% of the original amount of chlorosulfonic acid charged to the reactor. Quenching unreacted chlorosulfonic acid and the heat of solution of hydrogen chloride were both exothermic and the reactor warmed several degrees after the water was added.

A nitrogen purge was established on the reactor, feeding subsurface into the reactor liquid/slurry. The reactor vent passed through a condenser, to a caustic scrubber for neutralization of hydrogen chloride, then to activated carbon beds for removal of residual volatile organics. Full cooling was maintained on the reactor during the reactor purge.

The 3,5-di-tert-butylbenzenesulfonic acid product was less dense than the dichloromethane solvent. After the purge step, agitation was stopped and the crude product layer was allowed to float to the top of the reaction mass. 180 kg of clear liquid was decanted from the bottom of the reactor to remove a portion of impurities and residual acid. 136 kg of fresh dichloromethane was added back to the reactor to re-slurry the product and allowed to cool to less than -20 °C.

The reactor was drained to a 0.6-m² HDPE filter equipped with a 40-90-µm HDPE frit applying a light 7-10 kPa vacuum beneath the filter frit.

408 kg of dichloromethane was charged to the reactor and cooled to - 26.5°C. One-third of the reactor contents is drained to the filter as a wash. The product was re-slurried on the filter then allowed to drain. This was repeated two more times until the reactor was empty and the product had been fully washed.

A room temperature nitrogen purge was set up on the filter to flow from the top of filter, through the wetcake, and out to a dry ice trap and activated carbon beds. The product was dried in this manner until the residual dichloromethane level was within specifications.

24.6 kg of 3,5-di-tert-butylbenzenesulfonic acid was produced at a purity of 97.4 wt% by ¹H NMR. The yield of 3,5-di-tert-butylbenzenesulfonic acid was 77.1 mol%. The amount of diallyl sulfonates present in the final product was 69 ppm.

### Inventive Example 4

The monohydrate of 3,5-di-tert-butylbenzenesulfonic acid was produced on a large-scale following Scheme 3 with exposure to anhydrous HCl under higher pressure conditions prior to reaction with the sulfonating agent chlorosulfonic acid.

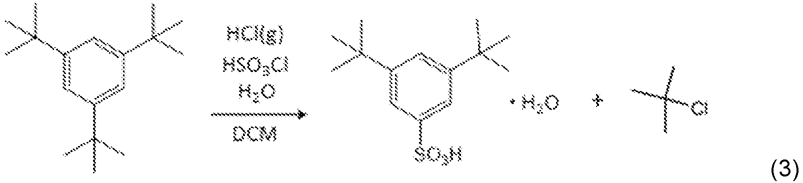

30 kg of 1,3,5-tri-tert-butylbenzene solid was charged to a nominal 200-gal glass-lined reactor. The reactor was then sealed, made inert with nitrogen, and then evacuated to 20 kPa (absolute) pressure. 255 kg of dichloromethane was pumped to the reactor. With agitation, full cooling was applied to the reactor through an external jacket. The reactor and its contents were cooled to -26°C. The 1,3,5-tri-tert-butylbenzene was fully soluble in dichloromethane at this temperature.

Gaseous, anhydrous hydrogen chloride was feed to the reactor from a cylinder, subsurface into the solution through a dip tube. Over a period of 6 hours, 14.3 kg anhydrous HCl was added to the reactor, increasing the reactor pressure to 123 kPa.

14.5 kg of chlorosulfonic acid was fed to the reactor, subsurface into the solution through a dip tube, over a period of 6 hours. The addition of chlorosulfonic acid was exothermic and the temperature of the reactor increased slightly. After the chlorosulfonic acid feed was complete, the reactor was allowed to cool down. During this period, a 3,5-di-tert-butylbenzenesulfonic acid precipitate formed in the reactor.

0.56 kg of deionized water was added to the reactor from the top of the reactor above the surface of the liquid to quench any unreacted chlorosulfonic acid. The 0.56 kg represented about 26% of the original amount of 1,3,5-tri-tert-butylbenzene charged to the reactor.

A nitrogen purge was established on the reactor, feeding subsurface into the reactor liquid/slurry. The reactor vent passed through a condenser, to a caustic scrubber for neutralization of hydrogen chloride, and then to activated carbon beds for removal of residual volatile organics. Full cooling was maintained on the reactor during the reactor purge.

Following the purge, an additional 1.7 kg of deionized water was added to the reactor in the same manner as before. This addition, with the 0.56 kg of deionized water added previously, was 105% of the initial 1,3,5-tri-tert-butylbenzene charged to the reactor, providing enough water to form the monohydrate of the 3,5-di-tert-butylbenzenesulfonic acid product. The reactor was allowed to cool after adding the second amount of deionized water.

The 3,5-di-tert-butylbenzenesulfonic acid monohydrate was less dense than the dichloromethane solvent. After the purge step, agitation was stopped and the crude product layer was allowed to float to the top of the reaction mass. 168 kg of clear liquid was decanted from the bottom of the reactor to remove a portion of impurities and residual acid. 265 kg of fresh dichloromethane was added back to the reactor to re-slurry the product and allowed to cool to less than -20°C.

The reactor was drained to a 0.6-m² HDPE filter equipped with a 40-90-µm HDPE frit applying a light 7-10 kPa vacuum beneath the filter frit.

539 kg of dichloromethane was charged to the reactor and cooled to -26.5°C. One-third of the reactor contents was drained to the filter as a wash. The product was re-slurried on the filter then allowed to drain. This was repeated two more times until the reactor was empty and the product had been fully washed.

A room temperature nitrogen purge was set up on the filter to flow from the top of filter, through the wetcake, and out to a dry ice trap and activated carbon beds. The product was dried in this manner until the residual dichloromethane was within specifications.

19.1 kg of 3,5-di-tert-butylbenzenesulfonic acid monohydrate was produced at a purity of 84.0 wt%, as determined by ¹H NMR. The major impurities were water and sulfuric acid. The yield of 3,5-di-tert-butylbenzenesulfonic acid was 50.0 mol%. No diallyl sulfonates were detected in the final product.

It is intended that the invention not be limited to the particular embodiment disclosed as the best mode contemplated for carrying out this invention, but that the invention will include all embodiments falling within the scope of the appended claims.

## Claims

1. A process for producing 3,5-di-tert-butylbenzenesulfonic acid comprising:
forming a solution of 1,3,5-tri-tert-butyl benzene in a solvent;
exposing said solution to anhydrous hydrogen chloride gas;
reacting 1,3,5-tri-tert-butylbenzene with a sulfonating agent to form 3,5-di-tert-butylbenzenesulfonic acid;
wherein said exposing of said solution to anhydrous hydrogen chloride gas is carried out before reacting said 1,3,5-tri-tert-butylbenzene with said sulfonating agent; and recovering said 3,5-di-tert-butylbenzenesulfonic acid.

2. The process of claim 1 wherein said solvent is selected to provide solubility for the amount of 1,3,5-tri-tert-butyl benzene used.

3. The process of claim 1 wherein said solvent is selected to provide solubility for the amounts of 1,3,5-tri-tert-butyl benzene and said sulfonating agent used.

4. The process of claim 1 wherein said solvent is selected to provide that 3,5-di-tert-butylbenzenesulfonic acid is substantially insoluble in said solvent.

5. The process of claim 4 wherein said 3,5-di-tert-butylbenzenesulfonic acid is recovered by filtration.

6. The process of claim 1 wherein said solvent is an organic solvent.

7. The process of claim 6 wherein said organic solvent is dichloromethane.

8. The process of claim 1 wherein said sulfonating agent is chlorosulfonic acid or sulfur trioxide.

9. The process of claim 1 wherein said exposing said solution to anhydrous hydrogen chloride gas and said reacting said 1,3,5-tri-tert-butyl benzene with a sulfonating agent is carried out at a temperature in the range of about -50°C to about 10°C.

10. The process of claim 1 wherein said exposing said solution to anhydrous hydrogen chloride gas is carried out under pressure.

11. The process of claim 1 wherein said exposing said solution to anhydrous hydrogen chloride gas is carried out to substantially saturate said solution with anhydrous hydrogen chloride.

12. The process of claim 1 wherein said exposing said solution to anhydrous hydrogen chloride gas is carried out by injecting said anhydrous hydrogen chloride gas into said solution.

## Patentansprüche

1. Prozess zum Herstellen von 3,5-Di-tert-butylbenzolsulfonsäure, umfassend:
Bilden einer Lösung von 1,3,5-Tri-tert-butylbenzol in einem Lösungsmittel;
Aussetzen der Lösung gegenüber wasserfreiem Chlorwasserstoffgas;
Umsetzen von 1,3,5-Tri-tert-butylbenzol mit einem Sulfonierungsmittel zum Bilden von 3,5-Di-tert-butylbenzolsulfonsäure;
wobei das Aussetzen der Lösung gegenüber wasserfreiem Chlorwasserstoffgas vor Umsetzen des 1,3,5-Tri-tert-butylbenzols mit dem Sulfonierungsmittel durchgeführt wird; und Gewinnen der 3,5-Di-tert-butylbenzolsulfonsäure.

2. Prozess nach Anspruch 1, wobei das Lösungsmittel so ausgewählt wird, dass es Löslichkeit für die verwendete Menge an 1,3,5-Tri-tert-butylbenzol vorsieht.

3. Prozess nach Anspruch 1, wobei das Lösungsmittel so ausgewählt wird, dass es Löslichkeit für die verwendeten Mengen an 1,3,5-Tri-tert-butylbenzol und des Sulfonierungsmittels vorsieht.

4. Prozess nach Anspruch 1, wobei das Lösungsmittel so ausgewählt wird, dass es vorsieht, dass 3,5-Di-tert-butylbenzolsulfonsäure in dem Lösungsmittel im Wesentlichen unlöslich ist.

5. Prozess nach Anspruch 4, wobei die 3,5-Di-tert-butylbenzolsulfonsäure durch Filtration gewonnen wird.

6. Prozess nach Anspruch 1, wobei das Lösungsmittel ein organisches Lösungsmittel ist.

7. Prozess nach Anspruch 6, wobei das organische Lösungsmittel Dichlormethan ist.

8. Prozess nach Anspruch 1, wobei das Sulfonierungsmittel Chlorsulfonsäure oder Schwefeltrioxid ist.

9. Prozess nach Anspruch 1, wobei das Aussetzen der Lösung gegenüber wasserfreiem Chlorwasserstoffgas und das Umsetzen des 1,3,5-Tri-tert-butylbenzols mit einem Sulfonierungsmittel bei einer Temperatur im Bereich von etwa -50°C bis etwa 10°C ausgeführt wird.

10. Prozess nach Anspruch 1, wobei das Aussetzen der Lösung gegenüber wasserfreiem Chlorwasserstoffgas unter Druck ausgeführt wird.

11. Prozess nach Anspruch 1, wobei das Aussetzen der Lösung gegenüber wasserfreiem Chlorwasserstoffgas so ausgeführt wird, dass die Lösung im Wesentlichen mit wasserfreiem Chlorwasserstoff gesättigt wird.

12. Prozess nach Anspruch 1, wobei das Aussetzen der Lösung gegenüber wasserfreiem Chlorwasserstoffgas durch Einspritzen des wasserfreien Chlorwasserstoffgases in die Lösung ausgeführt wird.

## Revendications

1. Processus de production d'acide 3,5-di-tert-butylbenzènesulfonique comprenant :
la formation d'une solution de 1,3,5-tri-tert-butylbenzène dans un solvant ;
l'exposition de ladite solution à du gaz chlorhydrique anhydre ;
la réaction du 1,3,5-tri-tert-butylbenzène avec un agent de sulfonation pour former de l'acide 3,5-di-tert-butylbenzènesulfonique ;
dans lequel ladite exposition de ladite solution au gaz chlorhydrique anhydre est effectuée avant la réaction dudit 1,3,5-tri-tert-butylbenzène avec ledit agent de sulfonation ; et la récupération dudit acide 3,5-di-tert-butylbenzènesulfonique.

2. Processus selon la revendication 1, dans lequel ledit solvant est choisi pour assurer la solubilité de la quantité de 1,3,5-tri-tert-butylbenzène utilisée.

3. Processus selon la revendication 1, dans lequel ledit solvant est choisi pour assurer la solubilité des quantités de 1,3,5-tri-tert-butylbenzène et dudit agent de sulfonation utilisées.

4. Processus selon la revendication 1, dans lequel ledit solvant est choisi de manière à ce que l'acide 3,5-di-tert-butylbenzènesulfonique soit sensiblement insoluble dans ledit solvant.

5. Processus selon la revendication 4, dans lequel ledit acide 3,5-di-tert-butylbenzènesulfonique est récupéré par filtration.

6. Processus selon la revendication 1, dans lequel ledit solvant est un solvant organique.

7. Processus selon la revendication 6, dans lequel ledit solvant organique est le dichlorométhane.

8. Processus selon la revendication 1, dans lequel ledit agent de sulfonation est l'acide chlorosulfonique ou le trioxyde de soufre.

9. Processus selon la revendication 1, dans lequel ladite exposition de ladite solution à du gaz chlorhydrique anhydre et ladite réaction dudit 1,3,5-tri-tert-butylbenzène avec un agent de sulfonation sont réalisées à une température dans la plage d'environ -50 °C à environ 10 °C.

10. Processus selon la revendication 1, dans lequel ladite exposition de ladite solution à du gaz chlorhydrique anhydre est effectuée sous pression.

11. Processus selon la revendication 1, dans lequel ladite exposition de ladite solution à du gaz chlorhydrique anhydre est effectuée pour saturer sensiblement ladite solution avec du chlorure d'hydrogène anhydre.

12. Processus selon la revendication 1, dans lequel ladite exposition de ladite solution au gaz chlorhydrique anhydre est réalisée en injectant ledit gaz chlorhydrique anhydre dans ladite solution.
